# EUROPÄISCHE PATENTSCHRIFT

(11) **EP 0 702 007 B1**
(45) Veröffentlichungstag und Bekanntmachung des Hinweises auf die Patenterteilung: **03.03.1999**
(21) Anmeldenummer: 95112629.1
(22) Anmeldetag: 11.08.1995
(51) Int. Cl.: C07D 239/96, C07B 41/10

(54) **Verfahren zur Herstellung von Chinazolin-2,4-dionen**
Process for the preparation of quinazoline-2,4-diones
Procédé de préparation de quinazolines-2,4-diones

(30) Priorität: 24.08.1994 DE 4429978
(43) Veröffentlichungstag der Anmeldung: 20.03.1996
(73) Patentinhaber: BAYER AG, 51368 Leverkusen (DE)
(72) Erfinder: Müller, Nikolaus, Dr., D-40789 Monheim (DE); Paetz, Klaus-Christian, Dr., D-51399 Burscheid (DE); Kiel, Wolfgang, Dr., D-51519 Odenthal (DE)

(56) Entgegenhaltungen:
- EP-A- 0 040 793
- EP-A- 0 176 333
- EP-A- 0 183 458
- EP-A- 0 260 817
- EP-A- 0 545 206
- EP-A- 0 653 423
- DE-A- 3 712 782
- EUROP.J.MED.CHEM., Bd. 9, Nr. 3, 1974 PARIS, Seiten 263-268, LESPAGNOL,A. ET AL. 'Dérivés de la benzoylène-urée'
- J.HETEROCYCLIC.CHEM., Bd. 19, 1982 Seiten 269-273, PAPADOPOULOS,E.P. ET AL. 'Convenient Preparation of N-substituted 2-Amino- 4H-3,1-benzoxazin-4-ones and 3-Substituted 2,4(1H,3H)-Quinazolinediones '
- CHEMICAL ABSTRACTS, vol. 83, no. 11, 15.September 1975 Columbus, Ohio, US; abstract no. 97348u, YABUUCHI,T. ET AL. 'Quinazolinedione Derivatives ' Seite 596; & JP-A-50 014 689 (RESEARCH INSTITUTE FOR PRDOCUTION DEVELOPMENT) 15 Februar 1975
- : "", J.ORG.CHEM., , , Band 26, Nr. , Seiten 5238 -

## Beschreibung

Die vorliegende Erfindung betrifft ein verbessertes Verfahren zur Herstellung von Chinazolin-2,4-dionen.

Es sind schon Verfahren zur Herstellung von Chinazolin-2,4-dionen bekannt. So kann man gegebenenfalls substituierte Anthranilsäuren mit einem Isocyanat zu einem Derivat umsetzen, bei dem die Aminogruppe der Anthranilsäure Teil eines Harnstoffs geworden ist und diesen Harnstoff unter Wasserabspaltung in Gegenwart von Ethanol cyclisieren (EP-A1 183 458). Nachteilig bei diesem Verfahren ist, daß es in zwei getrennten Stufen mit Zwischenisolierung des Harnstoffs durchgeführt werden muß, weil die Herstellung des Harnstoffs und die Cyclisierung in unterschiedlichen Lösungsmitteln erfolgen (siehe Beispiel 2 der EP-A 1 183 458).

Zur Herstellung von Chinazolin-2,4-dionen kann man auch Alkalisalze von Anthranilsäure mit Dithiocarbamaten umsetzen (siehe Synthesis 1983, 406). Nachteilig bei diesem Verfahren ist die Freisetzung leicht-flüchtiger organischer Schwefelverbindungen unter Einsatz des Hilfsmittels Quecksilberoxid. Bei einer technischen Durchführung dieses Verfahrens ist deshalb besonders umfangreicher Aufwand für Umweltschutzmaßnahmen erforderlich.

Weitere bekannte Verfahren zur Herstellung von Chinazolin-2,4-dionen und ähnlichen Verbindungen gehen von Anthranilsäureestern aus, die mit Isocyanaten umgesetzt werden (siehe Eur. J. Med. Chem. 9, 236 (1974), EP-A 260 817, C.A.83:97348 u (1975) und J. Org. Chem. 26, 25238 (1961)) oder von Anthranilsäuren, die mit Isocyanaten umgesetzt werden, wobei das als Zwischenprodukt entstehende Harnstoffderivat isoliert und in einer zweiten Stufe ähnlich der EP-A 183 458 zyklisiert wird (siehe EP-A 40 793) oder von Anthranilsäuren, die mit anorganischen Isocyanaten umgesetzt werden (siehe EP-A 176 333) oder von anderen Estern als Anthranilsäureester, wobei Uracilderivate erhalten werden (siehe DE-A 3 712 782) oder von β-Enaminoestern, die mit Isocyanaten zu Tetrahydro-pyrimidin-2,4-dionen umgesetzt werden (siehe EP-A 545 206).

Es wurde nun ein Verfahren zur Herstellung von Chinazolin-2,4-dionen der Formel (I) gefunden 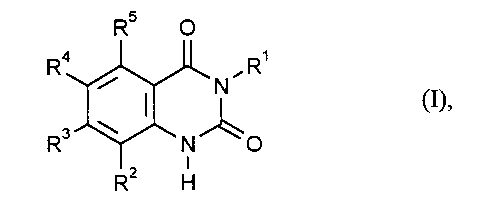 in der
- R¹: für Phenyl steht, das unsubstituiert oder mit 1 bis 3 Fluor- und/oder Chloratomen substituiert ist und
- R², R³, R⁴ und R⁵: unabhängig voneinander jeweils für Wasserstoff, Halogen, C₁-C₆-Alkyl oder C₁-C₆-Alkoxy stehen, wobei mindestens zwei der Reste R² bis R⁵ für Wasserstoff und höchstens zwei dieser Reste für Fluor, Chlor oder Brom stehen,
das dadurch gekennzeichnet ist, daß man eine Anthranilsäure der Formel (II) 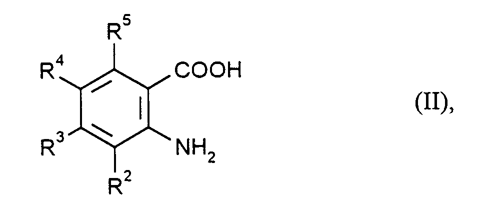 in der
- R², R³, R⁴ und R⁵: die bei Formel (I) angegebene Bedeutung haben,
in einem organischen, aprotischen Lösungsmittel mit einem Isocyanat der Formel (III) umsetzt

R¹-N=C=O (III),

in der
- R¹: die bei Formel (I) angegebene Bedeutung hat,
und anschließend ohne Isolierung des als Zwischenprodukt entstandenen Harnstoffs das Reaktionsgemisch mit Säure behandelt.

Halogen kann Fluor, Chlor, Brom und/oder Iod bedeuten, vorzugsweise bedeutet es Fluor, Chlor oder Brom.

Besonders bevorzugt setzt man 2-Amino-3-fluorbenzoesäure oder 2-Amino-5-fluorbenzoesäure und 2,4-Dichlorphenylisocyanat in das erfindungsgemäße Verfahren ein und stellt 3-(2,4-Dichlorphenyl)-8-fluor-chinazolin-2,4-dion oder 3-(2,4-Dichlorphenyl)-6-fluor-chinazolin-2,4-dion her.

Anthranilsäuren der Formel (II) sind bekannte Verbindungen oder analog zu bekannten Verbindungen zugänglich, beispielsweise durch katalytische Hydrierung der entsprechenden Nitrobenzoesäuren. Solche Nitrobenzoesäuren können z.B. gemäß Tetrahedron Letters 40, 5115 (1988) oder analog dazu hergestellt werden.

Die Isocyanate der Formel (III) sind ebenfalls bekannte Verbindungen oder analog zu bekannten Verbindungen zugänglich.

Bezogen auf 1 Mol Anthranilsäure der Formel (II) kann man beispielsweise von 0,8 bis 1,2 Mol eines Isocyanats der Formel (III) einsetzen. Vorzugsweise wurden äquimolare Mengen eingesetzt.

Als organische, aprotische Lösungsmittel für das erfindungsgemäße Verfahren kommen z.B. in Frage: Ester wie Essigsäuremethyl- und -ethylester, Lactone wie Butyrolacton, cyclische Ether wie Tetrahydrofuran und Dioxan, Amide wie Dimethylformamid und Dimethylacetamid, Lactame wie N-Methylpyrrolidon, Ketone wie Aceton, Methylisobutylketon und Cyclohexanon und aprotische organische Schwefelverbindungen wie Dimethylsulfoxid und Tetramethylensulfon.

Es ist vorteilhaft, das erfindungsgemäße Verfahren mindestens mit soviel Lösungsmittel durchzuführen, daß das Reaktionsgemisch stets rührbar ist.

Die Temperatur bei der Umsetzung einer Anthranilsäure der Formel (II) mit einem Isocyanat der Formel (III) kann beispielsweise zwischen 0 und 200°C betragen. Vorzugsweise liegt sie bei 20 bis 150°C. Wenn man ein Arbeiten unter Druck vermeiden möchte, dann setzt man Lösungsmittel ein, deren Siedepunkt bei Normaldruck bei oder über der gewünschten Reaktionstemperatur liegt.

Nach Abklingen der Reaktion zwischen der Anthranilsäure und dem Isocyanat ist es vorteilhaft noch einige Zeit, z.B. bei 20 bis 150°C, nachzurühren, z.B. 1/2 bis 5 Stunden.

Es ist ein wesentliches Merkmal der vorliegenden Erfindung, daß die Zugabe einer Säure ohne Isolierung des als Zwischenprodukt gebildeten Harnstoffs erfolgt. Vorzugsweise erfolgt die Säurezugabe in die gleiche Apparatur, in der die Umsetzung der Anthranilsäure mit dem Isocyanat durchgeführt wurde.

Als Säuren sind insbesondere starke organische und anorganische Säure geeignet, wie Sulfonsäuren, Schwefelsäure, Halogenwasserstoffsäure und Phosphorsäure. Bevorzugt sind Schwefelsäure und Salzsäure, besonders bevorzugt ist Schwefelsäure. Vorzugsweise kommen die Säuren in wasserarmer oder wasserfreier Form zum Einsatz.

Die Säure kann, bezogen auf ein Mol des Isocyanats der Formel (III), z.B. in einer Menge von 0,2 bis 5 Mol eingesetzt werden. Vorzugsweise beträgt diese Menge von 0,5 bis 3 Mol.

Die Einwirkung der Säure kann beispielsweise bei Temperaturen von 0 bis 200°C und für eine Zeitdauer von 0,5 bis 12 Stunden erfolgen. Bevorzugt sind Temperaturen von 20 bis 150°C und Einwirkungszeiten von 1 bis 8 Stunden.

Nach der Einwirkung der Säure liegt das hergestellte Chinazolin-2,4-dion im Reaktionsgemisch vor. Man kann das Reaktionsgemisch z.B. aufarbeiten, indem man es auf Raumtemperatur abkühlt, filtriert, den erhaltenen Filterkuchen mit dem verwendeten Lösungsmittel nachwäscht und anschließend bei mäßig erhöhter Temperatur im Vakuum trocknet.

Mit dem erfindungsgemäßen Verfahren kann man Chinazolin-2,4-dione der Formel (I) in hohen Ausbeuten (z.B. nahe 90 % d.Th.) und in hohen Reinheiten (z.B. über 98 %) erhalten. Weitere Reinigungsschritte sind im allgemeinen nicht notwendig.

Dies ist besonders überraschend, da in J. Heterocyclic Chem. 19, 269 (1982) beschrieben ist, daß die saure Kondensation von 2-(3-Arylureido)-benzoesäuren in guten Ausbeuten zu 3,1-Benzoxazin-4-onen führt, während die Einwirkung von wäßrig-alkoholischer Natronlauge in guten Ausbeuten Chinazolin-2,4-dione ergibt.

### Beispiele

### Beispiel 1

In einem Vierhalskolben mit Rührer, Rückflußkühler, Thermometer und Tropftrichter wurden 31 g 5-Fluoranthranilsäure in 280 ml Essigsäureethylester bei 50°C gelöst. Hierzu wurden innerhalb von 20 Minuten 37,6 g geschmolzenes 2,4-Dichlorphenylisocyanat aus einem beheizbaren Tropftrichter zugetropft. Die Innentemperatur stieg auf 65°C an, wobei sich das Reaktionsgemisch verdickte, aber noch rührbar blieb. Es wurde 2 Stunden am Rückfluß nachgerührt, dann auf 70°C abgekühlt und im Laufe von 10 Minuten 39,5 g konzentrierte Schwefelsäure zugetropft. Die Innentemperatur stieg wieder auf Rückflußtemperatur an. Bei dieser Temperatur wurde 4,5 Stunden lang nachgerührt, wobei eine gut rührbare Suspension entstand. Danach wurde auf Raumtemperatur abgekühlt, das Produkt abgesaugt und auf der Nutsche 2 x mit je 75 ml Essigsäureethylester nachgewaschen. Nach dem Trocknen wurden 59 g 3-(2,4-Dichlorphenyl)-6-fluorchinazolin-2,4-dion mit einem Schmelzpunkt von über 250°C, in einer Reinheit von 99 % und in einer Ausbeute von 89,8 % d.Th. erhalten.

### Beispiel 2

Analog der in Beispiel 1 beschriebenen Arbeitsweise wurden 136 g Anthranilsäure und 188 g 2,5-Dichlorphenylisocyanat in einem Liter Essigsäureethylester zum Harnstoff umgesetzt. In die siedende Suspension des (2-Carboxyphenyl)-(2',5'-Dichlorphenyl)-harnstoffs wurden innerhalb einer Stunde 36,5 g Chlorwasserstoffgas eingeleitet. Es wurde 3 Stunden am Rückfluß nachgerührt, auf Raumtemperatur abgekühlt und das ausgefallene Produkt durch Filtration isoliert. Nach dem Trocknen lagen 285 g reines 3-(2,5-Dichlorphenyl)-chinazolin-2,4-dion mit einem Schmelzpunkt von 228 bis 231°C vor. Das entspricht einer Ausbeute von 93 % d. Th.

### Beispiel 3

42,5 g 4-Chloranthranilsäure wurden in 250 ml N-Methylpyrrolidon gelöst und bei Raumtemperatur 29,5 g Phenylisocyanat in 30 Minuten zugetropft. Es wurde 5 Stunden bei 80°C nachgerührt und dann tropfenweise innerhalb von 1 Stunde mit 24,5 g konz. Schwefelsäure versetzt. Anschließend wurde bei 80°C 3 Stunden nachgerührt, dann auf Raumtemperatur abgekühlt, 500 ml Wasser zugefügt, das ausgefallene Produkt abgesaugt, mit Wasser gewaschen und im Vakuumtrockenschrank getrocknet. Es wurden so 57,4 g (85 % d. Th.) reines 3-Phenyl-7-chlor-chinozolin-2,4-dion mit einem Schmelzpunkt von über 300°C erhalten.

### Beispiel 4

21,6 g 5-Bromanthranilsäure und 15,4 g 4-Chlorphenylisocyanat wurden in analoger Arbeitsweise wie in Beispiel 1 beschrieben in 200 ml Butylacetat bei 100 °C und mit einer Nachrührzeit von 4 Stunden zur Reaktion gebracht. Danach wurde in die Suspension des gebildeten Harnstoffes 18 g Chlorwasserstoffgas innerhalb von 30 Minuten eingeleitet und anschließend bei 100°C 3 Stunden nachgerührt. Zur Aufarbeitung des Reaktionsgemisches wurde zunächst so viel Butylacetat bei Normaldruck abdestilliert, bis eine dicke Suspension entstanden war, dann mit 200 ml Wasser versetzt und das ausgefallene Produkt abfiltriert. Nach dem Trocknen wurden 31,5 g (90 % d. Th.) 6-Brom-3-(4-Chlorphenyl)-chinazolin-2,4-dion mit einem Schmelzpunkt von 212-214°C (Zersetzung) erhalten.

## Patentansprüche

1. Verfahren zur Herstellung von Chinazolin-2,4-dionen der Formel (I) 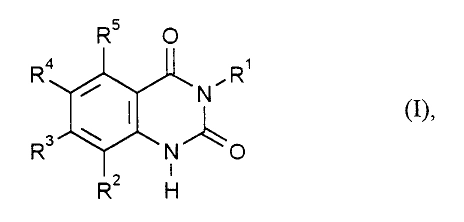 in der
R¹ für Phenyl steht, das unsubstituiert oder mit 1 bis 3 Fluor- und/oder Chloratomen substituiert ist und
R², R³, R⁴ und R⁵ unabhängig voneinander jeweils für Wasserstoff, Halogen, C₁-C₆-Alkyl oder C₁-C₆-Alkoxy stehen, wobei mindestens zwei der Reste R² bis R⁵ für Wasserstoff und höchstens zwei dieser Reste für Fluor, Chlor oder Brom stehen,
dadurch gekennzeichnet, daß man eine Anthranilsäure der Formel (II) 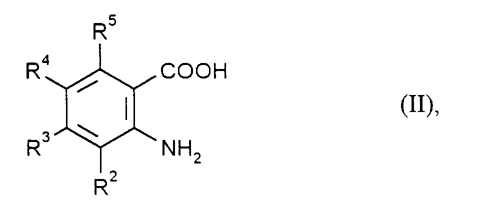 in der
R², R³, R⁴ und R⁵ die bei Formel (I) angegebene Bedeutung haben,
in einem organischen, aprotischen Lösungsmittel mit einem Isocyanat der Formel (III) umsetzt
R¹-N=C=O (III),
in der
R¹ die bei Formel (I) angegebene Bedeutung hat,
und anschließend ohne Isolierung des als Zwischenprodukt entstandenen Harnstoffs das Reaktionsgemisch mit Säure behandelt.

2. Verfahren nach Anspruch 1, dadurch gekennzeichnet, daß man bezogen auf 1 Mol Anthranilsäure der Formel (II) von 0,8 bis 1,2 Mol eines Isocyanats der Formel (III) einsetzt.

3. Verfahren nach Ansprüchen 1 bis 2, dadurch gekennzeichnet, daß man als Lösungsmittel Ester, Lactone, cyclische Ether, Amide, Lactame, Ketone oder aprotische organische Schwefelverbindungen einsetzt.

4. Verfahren nach Ansprüchen 1 bis 3, dadurch gekennzeichnet, daß man die Umsetzung einer Anthranilsäure der Formel (II) mit einem Isocyanat der Formel (III) bei Temperaturen zwischen 0 und 200°C durchführt.

5. Verfahren nach Ansprüchen 1 bis 4, dadurch gekennzeichnet, daß man nach dem Abklingen der Reaktion zwischen der Anthranilsäure und dem Isocyanat noch 1/2 bis 5 Stunden bei 20 bis 150°C nachrührt.

6. Verfahren nach Ansprüchen 1 bis 5, dadurch gekennzeichnet, daß man als Säuren eine Sulfonsäure, Schwefelsäure, eine Halogenwasserstoffsäure oder Phosphorsäure einsetzt.

7. Verfahren nach Ansprüchen 1 bis 6, dadurch gekennzeichnet, daß man bezogen auf ein Mol des Isocyanats der Formel (III) von 0,2 bis 5 Mol Säure einsetzt.

8. Verfahren nach Ansprüchen 1 bis 7, dadurch gekennzeichnet, daß man die Säure bei Temperaturen von 0 bis 200°C und für eine Zeitdauer von 0,5 bis 12 Stunden einwirken läßt.

9. Verfahren nach Ansprüchen 1 bis 8, dadurch gekennzeichnet, daß man das nach der Reaktion vorliegende Reaktionsgemisch aufarbeitet, indem man es abkühlt, filtriert, den erhaltenen Filterkuchen mit dem verwendeten Lösungsmittel nachwäscht und abschließend bei mäßig erhöhter Temperatur im Vakuum trocknet.

## Claims

1. Process for the preparation of quinazoline-2,4-diones of the formula (I) 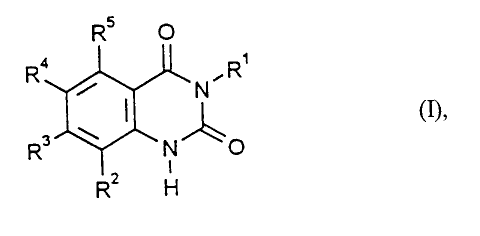 in which
R¹ represents phenyl which is unsubstituted or substituted by 1 to 3 fluorine and/or chlorine atoms and
R², R³, R⁴ and R⁵, independently of each other, each represent hydrogen, halogen, C₁-C₆-alkyl or C₁-C₆-alkoxy, at least two of the radicals R² to R⁵ representing hydrogen and at most two of these radicals representing fluorine, chlorine or bromine,
characterized in that an anthranilic acid of the formula (II) 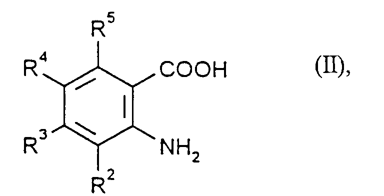 in which
R², R³, R⁴ and R⁵ have the meaning given under formula (I),
is reacted in an organic, aprotic solvent with an isocyanate of the formula (III)
R¹-N=C=O (III),
in which
R¹ has the meaning given under formula (I),
and then, without isolation of the urea formed as an intermediate, the reaction mixture is treated with acid.

2. Process according to Claim 1, characterized in that, based on 1 mol of anthranilic acid of the formula (II), from 0.8 to 1.2 mol of an isocyanate of the formula (III) are used.

3. Process according to Claims 1 to 2, characterized in that esters, lactones, cyclic ethers, amides, lactams, ketones or aprotic organic sulphur compounds are used as solvent.

4. Process according to Claims 1 to 3, characterized in that the reaction of an anthranilic acid of the formula (II) with an isocyanate of the formula (III) is carried out at temperatures between 0 and 200°C.

5. Process according to Claims 1 to 4, characterized in that, after the reaction between the anthranilic acid and the isocyanate has subsided, the mixture is stirred further at 20 to 150°C for an additional 1/2 to 5 hours.

6. Process according to Claims 1 to 5, characterized in that the acids used are a sulphonic acid, sulphuric acid, a hydrohalic acid or phosphoric acid.

7. Process according to Claims 1 to 6, characterized in that, based on one mol of the isocyanate of the formula (III), from 0.2 to 5 mol of acid are used.

8. Process according to Claims 1 to 7, characterized in that the acid is allowed to act at temperatures from 0 to 200°C and for a duration of 0.5 to 12 hours.

9. Process according to Claims 1 to 8, characterized in that the reaction mixture present after the reaction is worked up by cooling it, filtering it, washing the filter cake obtained with the solvent used and then drying it in vacuo at a moderately elevated temperature.

## Revendications

1. Procédé de préparation de quinazoline-2,4-diones de formule (I) : 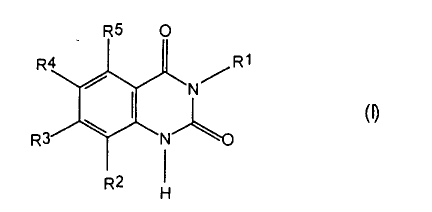
dans laquelle :
R¹ représente phényle qui est non substitué ou substitué par 1 à 3 atomes de fluor et/ou chlore, et
R², R³, R⁴ et R⁵ représentent indépendamment l'un de l'autre, hydrogène, halogène, alcoyle en C₁-C₆ ou alcoxy en C₁-C₆, où au moins deux des restes R² à R⁵ représentent hydrogène ou au moins deux de ces restes représentent fluor, chlore ou brome,
caractérisé en ce que l'on fait réagir un acide anthranilique de formule (II) : 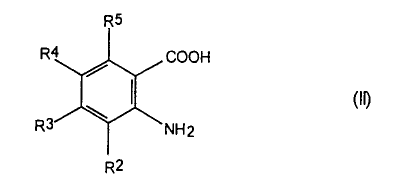
dans laquelle :
R², R³, R⁴ et R⁵ ont la signification donnée pour la formule (I),
dans un solvant organique, aprotique, avec un isocyanate de formule (III) :
R¹-N=C=O (III)
dans laquelle :
R¹ a la signification donnée pour la formule (I),
et ensuite, sans isolement de l'urée formée comme produit intermédiaire, on traite le mélange réactionnel avec un acide.

2. Procédé suivant la revendication 1, caractérisé en ce que l'on met en oeuvre, sur base de 1 mole d'acide anthranilique de formule (II), de 0,8 à 1,2 mole d'un isocyanate de formule (III).

3. Procédé suivant les revendications 1 à 2, caractérisé en ce que l'on met en oeuvre comme solvant, un ester, une lactone, un éther cyclique, un amide, un lactame, une cétone ou un composé soufré organique aprotique.

4. Procédé suivant les revendications 1 à 3, caractérisé en ce que l'on réalise la réaction d'un acide anthranilique de formule (II) avec un isocyanate de formule (III) à des températures comprises dans l'intervalle allant de 0 à 200°C.

5. Procédé suivant les revendications 1 à 4, caractérisé en ce que l'on poursuit l'agitation encore pendant 0,5 à 5 heures de 20 à 150°C, après arrêt de la réaction entre l'acide anthranilique et l'isocyanate.

6. Procédé suivant les revendications 1 à 5, caractérisé en ce que l'on met en oeuvre comme acide, un acide sulfonique, l'acide sulfurique, un acide halogénohydrique ou l'acide phosphorique.

7. Procédé suivant les revendications 1 à 6, caractérisé en ce que l'on met en oeuvre de 0,2 à 5 moles d'acide, sur base de 1 mole de l'isocyanate de formule (III).

8. Procédé suivant les revendications 1 à 7, caractérisé en ce que l'on laisse agir l'acide à des températures comprises dans l'intervalle allant de 0 à 200°C et pour une période de 0,5 à 12 heures.

9. Procédé suivant les revendications 1 à 8, caractérisé en ce que l'on traite le mélange réactionnel présent après la réaction par refroidissement, filtration, lavage du gâteau de filtration obtenu avec le solvant utilisé et ensuite, séchage sous vide à température modérément élevée.
